# FASCICULE DE BREVET EUROPEEN

(11) **EP 3 416 650 B1**
(45) Date de publication et mention de la délivrance du brevet: **19.08.2020**
(21) Numéro de dépôt: 17704789.1
(22) Date de dépôt: 17.02.2017
(51) Int. Cl.: A61K 31/56, A61P 35/00

(54) **CELASTROL ET SES DERIVES DANS LE TRAITEMENT DES TUMEURS ET PATHOLOGIES PRE-CANCEREUSES CUTANEES**
CELASTROL UND DERIVATE DAVON ZUR BEHANDLUNG VON TUMOREN UND PRÄKANZERÖSEN HAUTKRANKHEITEN
CELASTROL AND DERIVATIVES THEREOF FOR THE TREATMENT OF TUMOURS AND PRECANCEROUS DISEASES OF THE SKIN

(30) Priorité: 17.02.2016 FR 1651279
(43) Date de publication de la demande: 26.12.2018
(73) Titulaire: Pierre Fabre Médicament, 92100 Boulogne-Billancourt (FR)
(72) Inventeur: NGUYEN, Thien, 31180 Rouffiac-Tolosan (FR); PILLON, Arnaud, 31650 Saint Orens de Gameville (FR)
(74) Mandataire: Regimbeau
(86) Numéro de dépôt international: PCT/EP2017/053596
(87) Numéro de publication internationale: WO 2017/140834

(56) Documents cités:
- WO-A1-2009/026163
- WO-A2-2007/117466
- ABBAS SABIHA ET AL: "Preclinical studies of celastrol and acetyl isogambogic acid in melanoma.", CLINICAL CANCER RESEARCH, vol. 13, no. 22 Pt 1, 15 novembre 2007 (2007-11-15), pages 6769-6778, XP002762527, AN OFFICIAL JOURNAL OF THE AMERICAN ASSOCIATION FOR CANCER RESEARCH ISSN: 1078-0432, DOI: 10.1158/1078-0432.CCR-07-1536
- RADHAMANI KANNAIYAN ET AL: "Molecular targets of celastrol derived from Thunder of God Vine: Potential role in the treatment of inflammatory disorders and cancer", CANCER LETTERS, vol. 303, no. 1, 28 octobre 2010 (2010-10-28), pages 9-20, XP028152697, NEW YORK, NY, US ISSN: 0304-3835, DOI: 10.1016/J.CANLET.2010.10.025 [extrait le 2010-11-01]
- SATTAR E ABDEL ET AL: "QUINONE-METHIDE TRITERPENES FROM TISSUE CULTURES OF CATHA EDULIS", SAUDI PHARMACEUTICAL JOURNAL, vol. 6, no. 3-4, 1 juillet 1998 (1998-07-01), pages 242-245, XP008078000, SAUDI PHARMACEUTICAL SOCIETY, RIYAD, SA ISSN: 1319-0164
- CHEN YAN ET AL: "Formulation, characterization, and evaluation of in vitro skin permeation and in vivo pharmacodynamics of surface-charged tripterine-loaded nanostructured lipid carriers", INT. J. NANOMED., vol. 7, 2012, pages 3023-3033, XP002762528,
- MUNHOZ VANESSA MARQUITO ET AL: "Avaliação do fator de proteção solar em fotoprotetores acrescidos com extratos da flora brasileira ricos em substâncias fenólicas (Evaluation of the Sun Protection Factor in sunscreens added to with vegetable extracts rich in phenolic substances)", REVISTA DE CIENCIAS FARMACEUTICAS BASICA E APLICADA, vol. 33, no. 2, avril 2012 (2012-04), pages 225-232, XP002762529, ISSN: 1808-4532

## Description

La présente invention est définie par les revendications jointes ci-après. La présente invention concerne un triterpène, le Célastrol, encore nommé Triptérine, et ses dérivés et leurs sels pharmaceutiquement acceptables pour leur utilisation dans le traitement et la prévention de tumeurs et pathologies pré-cancéreuses cutanées par la voie topique.

Les tumeurs et pathologies pré-cancéreuses cutanées constituent un grave problème de santé publique. Un certain nombre d'entre elles sont causées par des expositions fréquentes aux rayons UV et peuvent évoluer vers des formes cancéreuses mortelles. On distingue essentiellement deux types de tumeurs cutanées :
1) Les carcinomes se développent à partir de cellules de l'épiderme, soit de la couche basale (carcinome basocellulaire), soit des couches supérieures (carcinome spinocellulaire ou carcinome épidermoïde cutané (appelé « *squamous cell carcinoma* » en anglais)). Ce sont les plus fréquents, puisqu'ils représentent 90 % des cancers de la peau, dont 75 % pour les basocellulaires et 20 % pour les spinocellulaires. Les carcinomes basocellulaires ne développent jamais de métastases; les spinocellulaires assez rarement, principalement dans les ganglions lymphatiques proches de la tumeur. Le développement des cancers suit généralement plusieurs étapes. Pour les carcinomes spinocellulaires, la tumeur débute souvent sous la forme d'une lésion localisée dans l'épiderme. En surface se forme une croûte (kératose actinique) ou une sorte d'eczéma (maladie de Bowen). C'est l'invasion du derme, plus profond, qui caractérise le stade de carcinome invasif.
2) Les mélanomes se développent à partir des mélanocytes qui sont les cellules qui fabriquent la mélanine, responsable de la pigmentation brune ou rouge de la peau. En effet, il existe deux types principaux de pigments : les bruns, qui confèrent le bronzage et une certaine protection contre les UV, et les rouges (peau rousse) qui ne protègent pas. Les sujets qui fabriquent essentiellement des pigments rouges ne bronzent pas et sont, de ce fait, plus à risque de cancers de la peau. Les mélanocytes sont normalement présents dans l'épiderme, associés aux cellules épidermiques de la partie profonde de l'épiderme. Le « grain de beauté », ou naevus, est une lésion bénigne qui correspond à une accumulation de mélanocytes dans le derme, ce qui explique sa couleur brune ou rouge. Les mélanomes sont beaucoup plus rares, mais peuvent se développer chez des sujets jeunes. Il faut les détecter et les traiter rapidement, car ils peuvent diffuser dans tout le corps et donner des métastases très difficiles à traiter.

La kératose actinique (KA) est une pathologie dermatologique encore appelée kératose solaire. C'est une hypertrophie des couches cornées de l'épiderme. La KA est le premier stade conduisant au développement du carcinome épidermoïde et est donc considérée comme une lésion « précancéreuse ». Bien que la grande majorité des KA soient bénignes, certaines études rapportent qu'environ 10% peut se transformer en carcinome épidermoïde. 40 à 60% des carcinomes épidermoïdes proviennent de KA non traitées. Environ 2 à 10% des carcinomes épidermoïdes s'étendent à des organes internes et menacent le pronostic vital.

La KA survient aux endroits du corps qui sont souvent exposés au soleil (notamment les UV-B). Celle-ci touche principalement la population caucasienne. Par exemple, la prévalence de la kératose actinique en Australie est estimée à environ 40%. Aux Etats- Unis, on estime à plus de 8,2 millions les patients atteints de KA. Le coût annuel estimé pour traiter la KA aux Etats-Unis est environ 1 milliard $ US en 2002.

Différentes possibilités thérapeutiques existent pour le traitement de la KA : la cryothérapie, la chirurgie, la thérapie photodynamique topique (PDT), le laser, le curetage et l'électrocoagulation, les applications topiques contenant de l'imiquimod, du 5-fluorouracile, du mébutate d'ingénol ou du diclofénac. Le traitement est à adapter en fonction de la localisation de la KA et de ses caractéristiques.

Cependant, ces divers traitements sont parfois sans succès, certains sont mal tolérés par les patients et certains d'entre eux peuvent laisser des traces irréversibles et inesthétiques. Il existe donc toujours un réel besoin de traitements alternatifs, efficaces, bien tolérés et rapides de la KA.

Le Célastrol (CLS) de formule (I) ci-dessous et ses dérivés notamment de type quinone-methyde triterpène dont le Pristimérine de formule (II) ci-dessous et le 22béta-hydroxy tingénone de formule (III) ci-dessous, sont des molécules extraites à partir des plantes de la famille *Celastaceae,* en particulier à partir des racines de *Maytenus ilicifolia* ou de *Tripterygium hypoglaucom* mais aussi de *Tripterygium wilfordii* ou encore de Lei gong teng, la plus connue en terme d'utilisation dans la médecine traditionnelle chinoise.

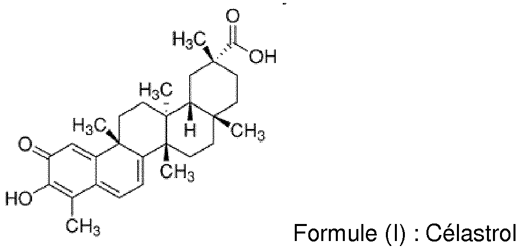

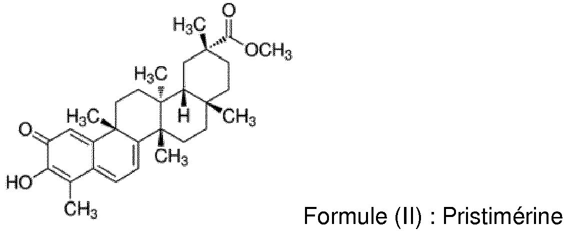

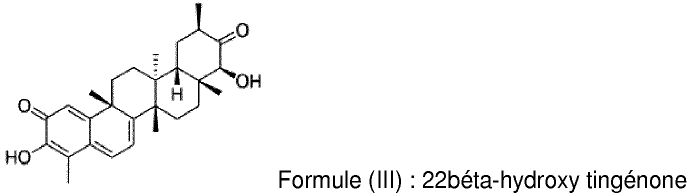

Les extractions du Célastrol et de ses dérivés à partir des racines de la plante sont souvent fastidieuses, coûteuses et non respectueuses du développement durable car étant des méthodes destructives. La synthèse totale par la voie chimique a également été décrite (Camelio et al. JACS 2015, 137, 11864-867). La solution alternative a été de réaliser des cultures cellulaires en suspension (CCV) de cellules souches générées à partir des racines ou des feuilles de la plante. Récemment, Coppede *et col.* (Plant Cell Tiss Organ Cuit 2014 ; 118 :33-43) a pu obtenir à partir de culture de cellules de *M. ilicifolia* de plus de dix ans d'âge une quantité notable de Célastrol (0,304 mg/g de cellules sèches, concentration maximale obtenue au bout de 8 jours de culture) soit plus de 8,85 fois meilleure que par la méthode d'extraction à partir de la plante.

Les inventeurs ont de façon surprenante mis en évidence l'efficacité du célastrol dans la prévention et le traitement de tumeurs ou de pathologies pré-cancéreuses cutanées, en particulier de la kératose actinique ou du carcinome épidermoïde cutané (SCC).

La présente invention porte donc sur un composé sélectionné parmi le Célastrol, le Pristimérine, le 22béta-hydroxy tingénone et les sels pharmaceutiquement acceptables de ceux-ci, et leurs mélanges pour son utilisation dans le traitement et/ou la prévention par voie topique d'une kératose actinique.

La présente invention porte, en outre, sur une composition topique comprenant au moins un composé selon l'invention, et au moins un excipient pharmaceutiquement acceptable pour son utilisation dans le traitement et/ou la prévention d'une kératose actinique.

Dans la présente invention, on entend désigner par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

On entend désigner par « sel pharmaceutiquement acceptable » d'un composé, un sel qui est pharmaceutiquement acceptable, comme défini ici, et qui possède l'activité pharmacologique souhaitée du composé parent.

Les sels pharmaceutiquement acceptables comprennent notamment :
(1) les sels d'addition d'acide pharmaceutiquement acceptable formés avec des acides inorganiques pharmaceutiquement acceptables tels que l'acide chlorhydrique, l'acide bromhydrique, l'acide sulfurique, l'acide nitrique, l'acide phosphorique et similaires ; ou formés avec des acides organiques pharmaceutiquement acceptables tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p- toluènesulfonique, l'acide triméthylacétique, l'acide trifluoroacétique et similaires, et
(2) les sels d'addition de base pharmaceutiquement acceptable formés
lorsqu'un proton acide présent dans le composé parent est soit remplacé par un ion métallique, par exemple un ion de métal alcalin, un ion de métal alcalino-terreux ou un ion d'aluminium ; soit coordonné avec une base organique pharmaceutiquement acceptable telle que la diéthanolamine, l'éthanolamine, N-méthylglucamine, la triéthanolamine, la trométhamine et similaires ; ou avec une base inorganique pharmaceutiquement acceptable telle que l'hydroxyde d'aluminium, l'hydroxyde de calcium, l'hydroxyde de potassium, le carbonate de sodium, l'hydroxyde de sodium et similaires.

Il pourra s'agir d'un sel de sodium lorsque le composé comporte une fonction acide.

Par « dérivé du Célastrol » on entend tous dérivés du Célastrol présentant une efficacité équivalente ou supérieure à celui-ci. L'efficacité du Célastrol et de ses dérivés pourra notamment être mesurée par l'évaluation de l'effet de ceux-ci sur la variation du nombre de lésions de kératose actinique lors d'un test d'efficacité tel que notamment celui présenté dans les exemples ci-dessous. Par « efficacité équivalente », on entend que le dérivé du Célastrol aura une efficacité comprise entre 50 et 100%, préférentiellement entre 80 et 100% de celle du Célastrol. Par conséquent, par « efficacité équivalente ou supérieure », on entend que le dérivé du Célastrol aura une efficacité supérieure ou égale à 50%, préférentiellement 80%, de celle du Célastrol.

Dans un mode de réalisation, les dérivés du Célastrol sont des métabolites terpénoïdes actifs tels que notamment ceux cités dans Brinker et al. (2007) Phytochemistry 68:732-766 ; Tang WJ et al. Eur J Med Chem 2015, 95:166-173 ; Luo DQ et al. Pest Manag Sci 2005, 61(1):85-90 ;et Morita H et al. Bioorg & Med Chem Let 2008, 18 :1050-52. Le dérivé du Célastrol pourra être le Pristimérine ou le 22béta-hydroxy tingénone. Préférentiellement, le dérivé du Célastrol sera le Pristimérine.

Dans un autre mode de réalisation le composé selon l'invention est sélectionné dans le groupe comprenant ou consistant en le Célastrol, le Pristimérine, le 22béta-hydroxy tingénone et un de leurs sels pharmaceutiquement acceptables. Dans un autre mode de réalisation le composé selon l'invention est sélectionné dans le groupe comprenant ou consistant en le Célastrol, le Pristimérine et un de leurs sels pharmaceutiquement acceptables. Dans un autre mode de réalisation le composé selon l'invention est le Célastrol ou un de ses sels pharmaceutiquement acceptable.

Le Célastrol, ses dérivés et leurs sels pharmaceutiquement acceptable selon l'invention peuvent être obtenu de toutes les façons connues par l'homme du métier.

Les compositions selon l'invention peuvent contenir du Célastrol et/ou un ou plusieurs de ses dérivés et/ou leur sels pharmaceutiquement acceptable sous forme purifiée ou sous forme d'extrait, qui peut être par exemple brut ou enrichi.

Par « forme purifiée », on entend, au sens de la présente invention, un composé comprenant au moins 90% en poids, notamment au moins 95% en poids, notamment au moins 99% en poids de Célastrol, d'un de ses dérivés ou d'un sel pharmaceutiquement acceptable de ceux-ci. Le Célastrol pourra notamment être obtenu par synthèse chimique notamment telle que décrite dans Camelio et col. (JACS 2015, 137 :11864-867).

Dans un autre mode de réalisation, le Célastrol, ses dérivés et leurs sels pharmaceutiquement acceptables selon l'invention peuvent être sous forme d'extrait après extraction de plantes de la famille *Celastaceae,* notamment de plantes telles que *Tripterygium wilfordii, Tripterygium regeli, Tripterygium hypoglaucom, Celastrus orbiculatus ou Maytenus ilicifoli.* Cet extrait peut être brut ou enrichi en Célastrol, en un de ses dérivés, et/ou en un de leurs sels pharmaceutiquement acceptables. L'extrait peut alors comprendre éventuellement des composants (dont des composants actifs) issus de la plante autres que le Célastrol, ses dérivés et leurs sels pharmaceutiquement acceptables. L'« extrait brut » est l'extrait directement obtenu à partir des plantes. Par « extrait enrichi », on entend notamment un extrait dans lequel la quantité de Célastrol, d'un de ses dérivés, et/ou d'un de leurs sels pharmaceutiquement acceptables est supérieure à 30% en poids, notamment supérieur à 50% en poids par rapport à un extrait brut.

Les extraits purifiés, bruts ou enrichis selon l'invention peuvent être obtenus à partir de n'importe quelle partie des plantes de la famille *Celastaceae,* notamment les racines. Alternativement, ils peuvent être obtenus par des cultures de cellules végétales de ces plantes. Dans le cas de cultures de cellules végétales, cet extrait pourra notamment être obtenu à partir du surnageant, de la suspension ou de la biomasse desdites cultures cellulaires (tel que notamment décrit par Coppede *et col.* (Plant Cell Tiss Organ Cuit 2014 ; 118 :33-43)).

Dans un mode de réalisation, le composé selon l'invention, c'est-à-dire le Célastrol, un de ses dérivés ou un de leurs sels pharmaceutiquement acceptables, est sous forme purifiée.

Par « tumeur ou pathologie pré-cancéreuse cutanée », on entend en particulier les tumeurs ou pathologies pré-cancéreuses cutanées sélectionnées dans le groupe comprenant ou consistant en les tumeurs ou pathologies pré-cancéreuses UV-induites, les métastases et les lymphomes T cutanés, notamment les tumeurs ou pathologies pré-cancéreuses UV-induites.

Selon l'invention, la tumeur ou pathologie pré-cancéreuse cutanée UV-induite est la kératose actinique.

L'ensemble de ces tumeurs ou pathologies pré-cancéreuses cutanées est bien connu de l'Homme de l'art qui saura facilement les reconnaitre et les différencier les unes des autres.

Notamment, la kératose actinique pourra être caractérisée par l'apparition de lésions de kératose actinique. Par « lésion de kératose actinique », on entend une hypertrophie des couches supérieures de l'épiderme résultant en un épaississement de la couche cornée. Le stade initial se caractérise par l'apparition de taches érythémateuses aux limites imprécises, de taille variable (de l'ordre du centimètre) et de surface rugueuse. A un stade plus avancé, l'épaississement de la couche cornée s'accentue (hyperkératose jaune ou brunâtre, qui saigne si on essaie de la détacher). Habituellement le diagnostic est clinique, mais un examen histologique (biopsie cutanée) permet de poser un diagnostic définitif (par la présence d'hyperkératose ortho- et para-kératosique, d'un épiderme atrophique ou hyperacanthosique fait de kératinocytes dysplasiques avec nombreuses atypies nucléaires).

Des lésions microscopiques, uniquement visibles en histologie, précèdent l'apparition de lésions macroscopiques (visibles à l'œil nu).

Par « métastases » selon l'invention on entend les métastase de localisation cutanée de tout type de tumeur, par exemples les métastases des tumeurs du sein, du poumon, ou de mélanome.

Par « traitement » selon la présente invention, on entend l'inhibition de l'évolution, plus particulièrement la régression, préférentiellement la disparition de la tumeur ou pathologie précancéreuse cutanée selon l'invention.

Par « prévention » selon la présente invention, on entend empêcher ou retarder l'apparition de la tumeur ou pathologie précancéreuse cutanée selon l'invention.

Le traitement ou la prévention selon l'invention s'entend chez l'Homme ou l'animal. Lorsque la pathologie précancéreuse selon l'invention est la kératose actinique on entend en particulier par « traitement de la kératose actinique », l'inhibition de l'apparition de nouvelles lésions de kératose actinique, l'inhibition de l'évolution des lésions de kératose actinique existantes (notamment de microscopiques à macroscopiques), l'inhibition de la croissance des lésions de kératose actinique macroscopiques existantes, la régression des lésions de kératose actinique existantes, et/ou la disparition des lésions de kératose actinique existantes.

Par « topique » selon l'invention on entend que le composé ou la composition selon l'invention seront administrés par application à la surface de la peau ou des muqueuses.

La composition topique selon l'invention pourra notamment se présenter sous toutes les formes permettant une application topique : crème, gel, onguent, patch, etc. Préférentiellement, la composition selon l'invention sera sous forme de crème.

La composition topique selon l'invention comprendra préférentiellement du célastrol, et/ou un ou plusieurs de ses dérivés et/ou leurs sels pharmaceutiquement acceptable et au moins un excipient pharmaceutiquement acceptable.

Le Célastrol est une molécule très lipophile, donc très faiblement soluble dans l'eau. Une simulation *in silico* avec un logiciel ACD/Labs version 12.02 « (Advance Chemical Development, INC.) » permet de prédire sa solubilité (selon Hansen C.M. J Paint Technol 1967 ;39 :511). En fonction du pH par exemple, Hansen et al. indique que pour le Célastrol la solubilité croît avec le pH. Le Célastrol présente donc une meilleure solubilité en milieu basique. La composition selon l'invention aura donc préférentiellement un pH supérieur à 7, préférentiellement supérieur à 9.

Dans un mode de réalisation, la composition topique selon l'invention comprend du Célastrol et/ou un ou plusieurs de ses dérivés et/ou leurs sels pharmaceutiquement acceptable à une concentration comprise entre 0,002% et 20% en poids par rapport au poids de la composition finale, par exemple comprise entre 0,005 et 5%, notamment entre 0,25 et 1%.

Dans un autre mode de réalisation, la composition topique selon l'invention comprend du Célastrol ou un sel pharamceutiquement acceptable à une concentration comprise entre 0,002% et 20% en poids par rapport au poids de la composition finale, par exemple comprise entre 0,005 et 5%, notamment entre 0,25 et 1%.

Dans un autre mode de réalisation le Célastrol et/ou un ou plusieurs de ses dérivés et/ou leurs sels pharmaceutiquement acceptable est administré à une dose comprise entre 3,5 µg et 3,5 mg/cm² de peau, par exemple entre 150 µg et 3000 µg/cm², par exemple entre 300 µg et 750 µg/cm².

Le composé ou la composition selon l'invention sera administrée 1 à plusieurs fois par jour, la durée du traitement pouvant être variable selon la gravité de la tumeur ou pathologie précancéreuse cutanée à traiter et sera facilement ajustable par l'Homme du métier ou le praticien. Dans un mode d'administration la composition ne sera pas administrée plus de 1, 2, 3, 4 ou 5 fois, par exemple plus de 3 fois.

Par « excipient pharmaceutiquement acceptable » selon l'invention, on entend un excipient compatible avec les autres ingrédients de la composition et qui ne produit aucun effet adverse, allergique ou autre réaction indésirable quand il est administré à un animal ou un humain.

Par « excipient » selon l'invention, on entend notamment un véhicule comme le PEG400, le DMSO, le pentylène glycol, l'acide linolénique, l'adipate de diéthyle, le 3-oxohexanoate d'éthyle, l'acide ricinoléique, l'octyldodécanol, l'acide linoléique, l'huile de sésame, les triglycérides caprique/caprylique, etc. ; un ou plusieurs tensioactifs, par exemple des macrogols, des hydroxy stéarates, des esters d'acides gras éthoxylés, des alcools gras éthoxylés, etc. ; un ou plusieurs solvants, comme par exemple l'octyldodécanol, ou le propylèneglycol dicaprylocaprate ; un ou plusieurs polymères hydrosolubles, par exemple la PVP, l'acide hyaluronique ou le hyaluronate de sodium ; un ou plusieurs épaississants comme par exemple les gommes naturelles ou hémi-synthétiques ; un ou plusieurs gélifiants, par exemple des carbomères ; une ou plusieurs charges minérales par exemple l'oxyde de zinc, du talc, ou des argiles ; un ou plusieurs émulsifiants tels que l'alcool cétéarylique ; un ou plusieurs conservateurs, par exemple le phénoxyéthanol ; un ou plusieurs antibactériens ; un ou plusieurs antiseptiques ; un ou plusieurs agents antioxydants, par exemple l'acétate de tocophérol ; un ou plusieurs chélatants, par exemple l'EDTA ; un ou plusieurs pigments ; un ou plusieurs parfums ; un ou plusieurs colorants ; un ou plusieurs agents d'ajustement du pH tels que des sels, des acides, des bases ; ou un mélange de ceux-ci.

La présente invention est illustrée par les exemples qui suivent.

### EXEMPLES

L'apoptose est un événement fondamental dans le développement cellulaire (prolifération). La visualisation de l'apoptose ainsi que le phénomène de cicatrisation qui s'ensuit reflète l'efficacité des médicaments dans le traitement des cellules précancéreuses et cancéreuses. Le Picato® (0,015% mébutate d'ingénol) est un produit présent sur le marché et indiqué dans le traitement de la kératose actinique. Le Picato® est connu pour induire la mort cellulaire par un effet nécrotique qui élimine rapidement des cellules mortes, suivi par une phase de cicatrisation. Pour pouvoir évaluer le pouvoir « apoptotique » du Célastrol sur les cellules de la peau (kératinocytes de l'épiderme), nous avons comparé l'effet apoptotique induit par l'application topique du Célastrol par rapport à celui d'un produit déjà présent sur le marché.

### Etude chez la souris - réaction cutanée sur peaux saines

### 1. Matériels et méthodes

### 1. 1. Souris

Les souris non consanguines sans poil de la souche SKH-1 sont les plus communément utilisées en recherche dermatologique préclinique. Ces souris non-pigmentées et immunocompétentes permettent des manipulations aisées de la peau, l'application d'agents topiques, l'exposition aux rayonnements UV ainsi que la visualisation facile de la réponse cutanée. La cicatrisation des plaies, les réponses photo biologiques aiguës et la carcinogenèse de la peau sont bien caractérisées sur cette souche de souris. En outre, les tumeurs induites chez ces souris ressemblent, tant au niveau moléculaire que morphologique, aux tumeurs malignes UV-induites de la peau chez l'homme. Dans cette étude sur peaux saines, des femelles SKH-1 (18 à 20 grammes), âgées de 6 à 8 semaines (Laboratoires Charles River) sont traitées de façon topique par les différents composés.

### 1. 2. Produits utilisés

### Produits de référence, mébutate d'ingénol

Le Picato® (0,015% mébutate d'ingénol), utilisé chez l'homme dans le traitement des lésions de kératose actinique, a été administré aux animaux (2 à 3 animaux par groupe) par voie topique, pendant 24 heures sous patch occlusif de 19 mm de diamètre, soit 2,83 cm² (*Hill Top Research*, USA). Le patch est chargé avec 200µl de Picato® en crème, soit 30µg de principe actif. La zone de peau traitée est délimitée par tatouage sur chaque souris et le patch est maintenu sur la souris à l'aide d'un film Tegaderm™ et de bandes adhésives Omnifix® et Elastoplast™. Le pansement occlusif est retiré au bout de 24 heures, et les animaux sont suivis cliniquement pendant 3 à 4 semaines.

### Célastrol

Le Célastrol (Euromedex) est formulé dans un véhicule constitué de 5% DMSO - 70% glycérol - 25% eau. Le pH est ajusté par ajout de soude autour de 8 - 8,5 pour solubiliser complètement la poudre. La concentration initiale est de 10 mg/ml puis des dilutions en série dans le véhicule permettent d'obtenir des concentrations de 5 et 2,5 mg/ml. Le Célastrol est appliqué par voie topique, pendant 30 heures sous patch occlusif de 19 mm de diamètre. Les patchs sont chargés avec 200µl des différentes solutions de Célastrol, soient 2000, 1000 ou 500µg de principe actif.

Comme contrôle placebo, un groupe supplémentaire est traité avec 200µl de véhicule seul. La zone de peau traitée est délimitée par tatouage sur chaque souris et le patch est maintenu sur la souris à l'aide d'un film Tegaderm™ et de bandes adhésives Omnifix® et Elastoplast™. Le pansement occlusif est retiré au bout de 30 heures, et les animaux sont suivis cliniquement pendant 3 à 4 semaines.

### 1. 3. Evaluation de la réaction cutanée aux traitements

### Suivi clinique

Les animaux sont pesés deux fois par semaine. La zone traitée de chaque animal est photographiée deux à trois fois par semaine afin de suivre l'évolution de la réaction cutanée et la cicatrisation.

### 2. Résultats

### 2.1 Suivi clinique

Les traitements topiques sont bien tolérés : le Picato® et le Célastrol n'ont aucun effet sur le poids des souris SKH-1. Les souris traitées se sont développées de la même façon que celles ayant reçues le véhicule seul. Ceci indique que ces produits, par cette voie d'administration et aux doses utilisées, sont fort bien tolérés par les animaux et qu'il n'y a donc pas de toxicité systémique.

### 2.2. Réactions cutanées aux traitements

### Etude 1 : effet du mébutate d'ingénol sur peau saine

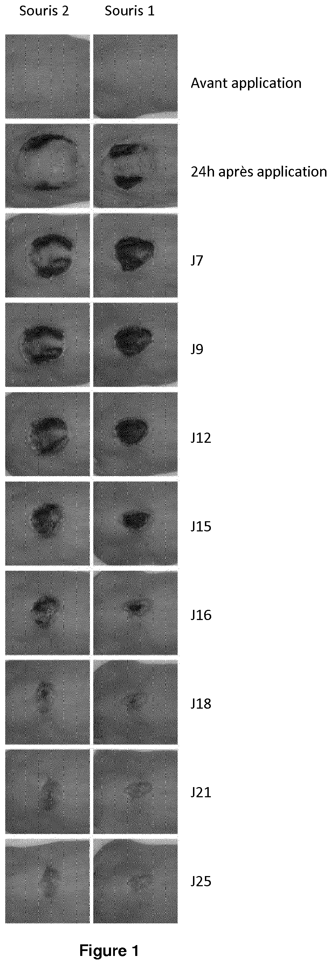

Une réaction cutanée au traitement par le mébutate d'ingénol est visible sur la peau des souris traitées dès le jour 2, soit 24h après l'arrêt du traitement occlusif (Figure 1). Cette réaction se manifeste par une phlyctène centrale, des rougeurs inflammatoires et des zones hémorragiques au niveau des marges de la zone traitée. L'ensemble de la zone lésée se nécrose à J7, ce qui reflète une forte activité de mort cellulaire (apoptose induite par le produit). La réaction cutanée semble profonde et les couches dermiques et hypodermiques sont touchées. Il y a la formation d'une croûte suivie d'une ré-épithélialisation à partir des berges de la plaie (à J12/J15). Puis la croûte se résorbe à J21 et la cicatrisation se prolonge (phase de maturation cicatricielle).

### Etude 2 : effet du Célastrol sur peau saine

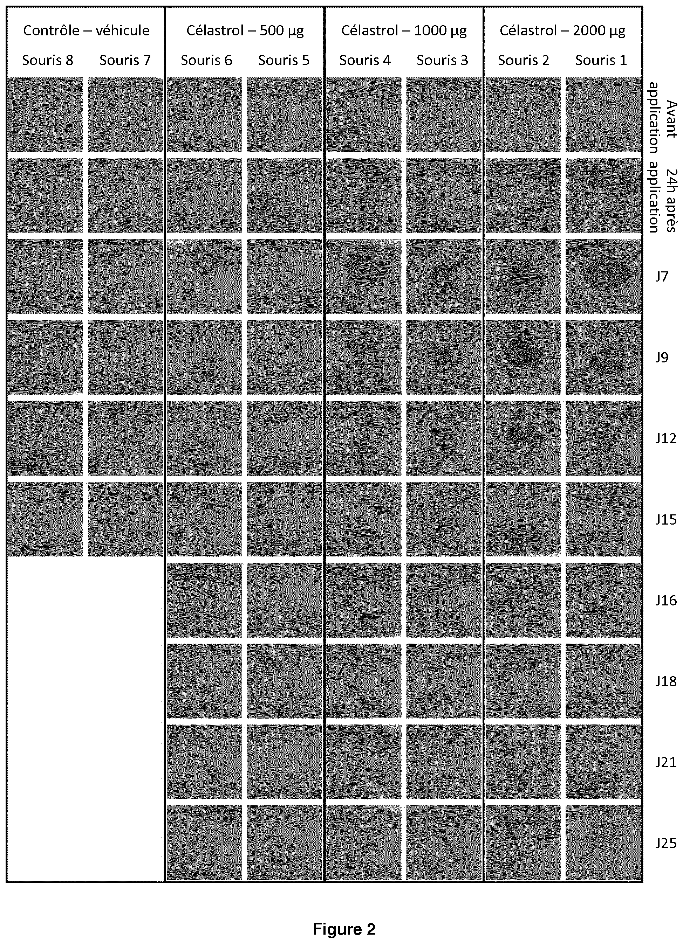

Une réaction cutanée au traitement par le Célastrol est visible sur la peau des souris traitées dès le jour 3, soit 24h après l'arrêt du traitement occlusif (Figure 2). Cette réaction dose dépendante se manifeste par une phlyctène centrale et des rougeurs inflammatoires. Contrairement à Picato®, on n'observe pas de zones hémorragiques même à la plus forte dose et l'action pro-apoptotique du Célastrol semble localisée au niveau de l'épiderme et reste superficielle. Le processus de cicatrisation démarre à J7 et de façon dose-dépendante, la peau retrouve un aspect quasi-normal quelques jours plus tard. Il reste une légère hypertrophie de la zone cicatricielle à J28 pour les doses de 1000 et 2000 µg. Le véhicule seul n'a aucun effet sur la peau des souris SKH-1.

De manière surprenante, il a donc été montré que sur la zone d'application de la peau de la souris, le Célastrol induit *in vivo* l'apoptose des cellules épidermiques de manière dose dépendante (à partir de 500µg/application) et ce comme également observé avec le Picato®. De plus, chez la souris traitée avec le Célastrol, environ une semaine après la phase d'apoptose, on observe une réparation cutanée par cicatrisation, la peau retrouve son aspect quasi-normal quelques jours plus tard. C'est la première fois que l'effet apoptotique *in vivo* du Célastrol a été démontré chez l'animal en application topique. Le Célastrol présente donc toutes les caractéristiques essentiellement pour un traitement efficace de pathologies pré-cancéreuses et cancéreuses, notamment de la kératose actinique : mort des cellules précancéreuses ou cancéreuses, régénération des nouvelles cellules pour revenir à la situation d'une peau saine.

### LEGENDES DES FIGURES

Figure 1 : Photographies de la peau de souris SKH-1 traitées par le Picato® (0,015% mébutate d'ingénol). Le pansement topique occlusif est maintenu pendant 24h et l'évolution de la réaction cutanée est ensuite suivie au cours du temps.
Figure 2 : Photographies de la peau de souris SKH-1 traitées par le Célastrol (2000, 1000 et 500 µg / souris, respectivement les souris représentatives 1&2, 3&4 et 5&6) par voie topique. Le groupe contrôle placebo est traité avec le véhicule seul (souris 7&8). Le pansement occlusif est maintenu pendant 30h et l'évolution de la réaction cutanée est ensuite suivie au cours du temps.

## Revendications

1. Composé sélectionné parmi le Célastrol, le Pristimérine, le 22béta-hydroxy tingénone et les sels pharmaceutiquement acceptables de ceux-ci, pour son utilisation dans le traitement et/ou la prévention par voie topique d'une kératose actinique.

2. Composition topique comprenant un ou plusieurs composé(s) selon la revendication 1, et au moins un excipient pharmaceutiquement acceptable pour son utilisation dans le traitement et/ou la prévention d'une kératose actinique.

3. Composition topique pour son utilisation selon la revendication 2, dans laquelle le composé est le Célastrol ou un sel pharmaceutiquement acceptable de celui-ci.

4. Composition topique pour son utilisation selon la revendication 2 ou 3, dans laquelle le ou les composé(s) est/sont présent(s) au sein d'un extrait obtenu d'une plante de la famille Celastaceae.

5. Composition topique pour son utilisation selon l'une quelconque des revendications 2 à 4, dans laquelle le ou les composé(s) est/sont présent(s) à une concentration comprise entre 0,002% et 20% en poids par rapport au poids de la composition finale.

6. Composition topique pour son utilisation selon l'une quelconque des revendications 2 à 5, dans laquelle le ou les composé(s) est/sont administré(s) à une dose comprise entre 3,5 µg/cm² et 3,5 mg/cm² de peau.

## Patentansprüche

1. Verbindung, ausgewählt aus Celastrol, Pristimerin, 22-Beta-Hydroxy-Tingenon und pharmazeutisch verträglichen Salzen davon, zu deren Verwendung in der Behandlung und/oder der Vorbeugung einer aktinischen Keratose auf topischem Wege.

2. Topische Zusammensetzung, die eine oder mehrere Verbindung(en) nach Anspruch 1 und mindestens einen pharmazeutisch verträglichen Hilfsstoff umfasst, zu deren Verwendung in der Behandlung und/oder der Vorbeugung einer aktinischen Keratose.

3. Topische Zusammensetzung zu deren Verwendung nach Anspruch 2, wobei es sich bei der Verbindung um Celastrol oder ein pharmazeutisch verträgliches Salz davon handelt.

4. Topische Zusammensetzung zu deren Verwendung nach Anspruch 2 oder 3, wobei die Verbindung (en) in einem Extrakt vorliegt/vorliegen, der von einer Pflanze aus der Familie der Celastaceae gewonnen wird.

5. Topische Zusammensetzung zu deren Verwendung nach einem der Ansprüche 2 bis 4, wobei die Verbindung(en), auf das Gewicht der fertigen Zusammensetzung bezogen, in einer Konzentration im Bereich zwischen 0,002 Gewichts-% und 20 Gewichts-% vorliegt/vorliegen.

6. Topische Zusammensetzung zu deren Verwendung nach einem der Ansprüche 2 bis 5, wobei die Verbindung(en) in einer Dosierung im Bereich zwischen 3,5 µg/cm² und 3,5 mg/cm² Haut verabreicht wird/werden.

## Claims

1. Compound selected from among celastrol, pristimerin, 22beta-hydroxy tingenone and pharmaceutically acceptable salts thereof, for use in the topical treatment and/or prevention of an actinic keratosis.

2. Topical composition comprising one or more compound(s) according to claim 1, and at least one pharmaceutically acceptable excipient for use in the treatment and/or the prevention of an actinic keratosis.

3. Topical composition for use according to claim 2, wherein the compound is celastrol or a pharmaceutically acceptable salt thereof.

4. Topical composition for use according to claim 2 or 3, wherein the compound(s) is/are present within an extract obtained from a plant of the Celastaceae family.

5. Topical composition for use according to any one of claims 2 to 4, wherein the compound(s) is/are present at a concentration of between 0.002% and 20% by weight with respect to the weight of the final composition.

6. Topical composition for use according to any one of claims 2 to 5, wherein the compound(s) is/are administered at a dose of between 3.5µg/cm² and 3.5mg/cm² of skin.
